# EUROPEAN PATENT SPECIFICATION

(11) **EP 2 903 546 B1**
(45) Date of publication and mention of the grant of the patent: **19.09.2018**
(21) Application number: 13844128.2
(22) Date of filing: 03.10.2013
(51) Int. Cl.: A61B 17/94, A61B 17/00

(54) **SURGICAL SUTURE SYSTEM, TISSUE RESTRAINT/SUTURE CAPTURE AND TISSUE ANCHOR**
CHIRURGISCHES NAHTSYSTEM, GEWEBEFIXIERER/NAHTERFASSUNG UND GEWEBEANKER
SYSTÈME DE SUTURE CHIRURGICALE, TISSU DE CONTENTION/SAISIE DE SUTURE ET ANCRAGE DE TISSU

(30) Priority: 04.10.2012 US 201261709481 P
(43) Date of publication of application: 12.08.2015
(73) Proprietor: Ziptek L.L.C., Sarasota, FL 34239 (US)
(72) Inventor: BENNETT, William, F., Sarasota, FL 34239 (US); GALAZ MENDEZ, Ramses, Hermosilo Mexico 83106 (MX); GOMEZ ROMO, Daniel, Francisco, Hermosillo Mexico 83106 (MX)
(74) Representative: Von Rohr Patentanwälte Partnerschaft mbB
(86) International application number: PCT/US2013/063277
(87) International publication number: WO 2014/055767

(56) References cited:
- EP-A1- 0 464 480
- WO-A1-2007/056583
- US-A- 5 725 556
- US-A- 6 015 428
- US-A1- 2012 101 526
- US-A1- 2012 101 526
- US-B1- 6 491 714

## Description

This invention relates generally to surgical apparatus and methods for repair of torn tissue, and more particularly to a system, components thereof, and method for arthroscopic and other surgical repair of tom tissue and tissue reattachment by providing the system for suturing and anchoring the tom tissue together against other tissue substrates, or for attaching tissue to medical, veterinary and dental implants.

In particular, the present invention relates to a surgical suture system according to the preamble of claim 1.

The rotator cuff is composed of four tendons that blend together to help stabilize and move the shoulder. When a tear occurs in the rotator cuff of the shoulder, it is often necessary to reattach the torn tendon or tendons to the bone of the humeral head. In a common prior art rotator cuff reattachment technique, the tom cuff is punctured by a punch, and prethreaded suture anchor screws (soft tissue fasteners) are drilled into the head of the humerus bone and the sutures threaded through the anchor screws are passed through the cuff in a difficult procedure using suture relay devices to pass the sutures through the tissue. After the suture strands are passed through the tissue, they are knotted and tied together to secure the reattached rotator cuff to the humerus head (knotted-technique). Other types of prior art suture anchors are cylindrically shaped members that are pressed into holes drilled into the bone and engage the cancellous mass surrounding the drilled hole and apply friction to hold the suture in place.

Many anchor/suture devices require knots to be tied, which is difficult with minimally invasive surgery and having a "knotless" solution is an advantage in these situations. It is desirable to have an engineered suture/capture construct that does not use friction to hold the suture in place, a technology employed and found in most of today's "knotless" tissue repair devices. Furthermore, a knotless device is desirable that does not crimp, crush, pinch or interfere with the suture with a friction type hold, which will avoid potential damage to the suture.

In U.S. Patent 6.491,714, an apparatus and method for arthroscopic repair of torn tissue such as a rotator cuff was taught wherein torn tissue such as a rotator cuff is positioned on the bone exterior by a tissue grasper. A cannula is inserted through the skin substantially to the tom tissue. A drill guide is inserted into the cannula, a drill bit is inserted into the drill guide, and a hole is drilled through the torn tissue and completely through the bone. The drill bit is removed and an inner cannula is passed through the drill guide until its distal end is engaged on the torn tissue or alternatively passed through the hole until its distal end is at the far end of the drilled hole. A soft tissue anchor having expandable wings at its distal end and sutures secured to an eyelet at its proximal end is releasably connected to the distal end of a tubular deployment tool with the free ends of the sutures extending through the deployment tool.

The deployment tool is passed through the inner cannula and a hole is drilled until the expandable wings clear the far end of the hole, a sufficient distance to allow the wings to expand to a diameter larger than the diameter of the drilled hole. The deployment tool, inner cannula, drill guide and cannula are removed and tension is applied to the suture to engage the expanded wings of the anchor on the exterior surface of the bone surrounding the drilled hole. A button is run down on the sutures through the cannula and secured on the torn tissue by the sutures such that the tom tissue is secured to the bone and the sutures are anchored to the hard exterior surface of the bone by the expanded anchor.

Unlike conventional soft tissues anchors which are anchored in the cancellous bone mass beneath the near cortex of the bone, the '714 teaching in one embodiment provides a suture anchor which is engaged on the exterior of the far cortex of the bone and completely bypasses the cancellous bone mass. The cortex of the bone is much less susceptible to osteopenia than the cancellous interior of the bone. The sutures are passed through the tissue when the anchor is set, and thus the difficult procedural step and use of devices such as punches and suture relays to pass and tie the sutures through the torn tissue is eliminated.

Calibrated markings on the '714 deployment system allow for precise measurement of the far cortex and precise measurement of the depth of insertion and engagement of the anchor device on the far cortex, such that structures beyond the cortex are not violated, and the button hold-down feature eliminates the traditionally difficult arthroscopic tying techniques.

In another broader aspect of the 714 invention, the surgical apparatus includes any form of a tissue substrate anchor of a conventional well-known structure, an elongated suture member securable at its proximal end to the anchor, and a separate tom tissue retainer which lockably engages as desired along the length of the suture member. The suture member extends through the torn tissue from the anchor and the tissue substrate. The torn tissue retainer is movable along the length of the exposed portion of the suture member until it is tightly positioned against the tom tissue and automatically locked in that position by non-reversible lockable engagement with the suture member. A separate tissue gripping member formed preferably as a semi-flexible plate or disc having a substantially larger surface area than the tissue retainer is also provided for enhanced retention of the torn tissue in place against the outer surface of the tissue substrate.

Still another broad aspect of this 714 invention is directed to a surgical apparatus which includes an integrally formed tissue substrate anchor having an elongated suture member formed as a unit therewith. A separate disc-shaped retainer lockingly engages with the exposed distal end of the suture portion at any desired point along the suture interlocking portion. The tissue retainer is therefore moveable along the length of the exposed engaging members of the suture member for tightening the tissue layer against the tissue substrate. A tear such as that found within a torn meniscus may be reconnected utilizing this embodiment of the invention.

Currently, soft-tissue fixation products that utilize "knot-less" technology and screws rely on an "interference-fit" for holding power between the screw and bone. In general, non-screw anchors have a pullout strength near 200 newtons, and screws can have upwards of 400 newtons of pullout strength.

The present disclosure allows for the introduction of a revolutionary type of anchor for soft-tissue fixation to bone. Screws, as opposed to hook-type anchors, have the strongest pullout strength. Moreover, this disclosed technology will introduce its technology to screws, thereby maximizing pullout strength capabilities in this tissue repair scenario, a scenario, which presently, can have failure secondary to the pullout of the anchor, slipping of the suture, breakage of the suture secondary to the crimping effect of the friction technology or complete system displacement. Additionally this presently disclosed suture/capture construct will be introduced to other types of medical, veterinary and dental implants and, when multiple captures are used with one suture, tissue to tissue lock with the capture itself acting as a tissue anchor or restraint will be provided Specifically, one member of the suture will attach to screws to allow for a ratcheting of the suture member through the suture capture or retainer or suture anchor, thereby creating a very strong construct.

The traditional repair of soft tissue requires sutures to be passed through the tissue. A knot is tied, which holds the torn tissue together, allowing for healing. Minimally invasive surgical techniques are being utilized through "button-hole" size incisions. Surgery is performed with instruments that pass through cannulas (like drainage culverts or pipes). Knots that would be utilized for this type of repair are tied and must be slid down through these cannulas. This technique can be difficult, result inadequate repair strength, provide for poor tissue approximation, for some surgeons, it may result in an inability to proceed with a minimally invasive approach secondary to the advanced technical difficulty, and finally, can add significant operative time to surgical procedures. Based upon the present disclosure, a "pipe-line" of products will be created using knot-less, self-locking interface as a technology development platform for all types of torn medical, veterinary or dental tissue and for attaching all types of tissue to medical, veterinary or dental implants.

The foregoing examples of the related art and limitations related therewith are intended to be illustrative and not exclusive. Other limitations of the related art will become apparent to those skilled in the art upon a reading of the specification and a study of the drawings.

The EP 0 464 480 A1 teaches a suture anchor for securing a suture to body tissue in an arthroscopic surgical procedure.

This document discloses features being present in the preamble of claim 1. The invention is defined in claim 1. Further embodiments of the invention are defined in the dependent claims.

The WO 2007/056583 A1 is related to a device for remodeling the tongue, wherein one or more spaces or cavities can be formed in the tongue. In one embodiment, the cavities are closed by inserting a tethered soft tissue anchor into the tongue

The US 2012/0101526 A1 discloses a surgical suture system, suture, and tissue engaging member for tissue repair and reattachment of torn tissue to a tissue substrate, medical prosthesis or medical implant.

Object of the present invention is to provide a surgical suture system which is more versatile and/or reliable.

This object is achieved by a surgical suture system according to claim 1. Advantages embodiments are subject of the dependent claims.

The present disclosure is broadly directed to a surgical suture system for tissue repair and reattachment of torn tissue to a tissue substrate found in the medical, veterinary or dental domains, medical veterinary or dental prosthesis or medical veterinary, dental implants. The system includes an elongated flexible suture member having a plurality of longitudinally spaced protuberances along a length thereof and utilized in conjunction with a suture capture, which can be utilized alone or in plurality with one suture or in conjunction with any of a plurality of tissue engaging members such as suture tissue restraints, anchors, and medical, veterinary or dental implants, each including uniquely configured locking apertures sized to receive the suture member passed therethrough to allow longitudinal movement of the suture member in only one direction through the locking apertures for suture member tightening and retention. Tissue engaging anchors structured to securely receive one or more of the suture tissue restraints or suture captures either before or during a surgical procedure.

For the purposes of this disclosure, the terms "tissue restraint" or "suture capture", alone or combined as tissue restrain/suture capture means a disk-shaped device for one-way locking engagement with a flexible surgically implantable suture having spaced apart protuberances, preferably beaded. When referred to as a tissue restraint, the device is being used to restrain tissue by the tensioning of the suture between the device and another anchor or restraint means. When referred to as a "capture", the device is being secured to a tissue implant or anchor or surgically implantable device such as a pacemaker or prosthetic for tensionable locking engagement with a suture. The term "construct" refers to the combination of a tissue restrain/suture capture, a suture, and a medical, veterinary, or dental implant wherein the tissue restrain/suture capture and the anchor or implant are independent or secured together.

The locking aperture of the tissue restrain/suture capture provides for higher resistance on the exit side of the locking aperture and lower resistance on the entrance side of the locking aperture. The suture easily passes through the entrance to the locking aperture of the tissue restrain/suture capture and leaves through the exit side of the locking aperture, both allowing the suture to travel forward in a "Go" direction. The distinct design prevents the suture from travelling in reverse or pulled backward from the entrance to the locking aperture in a "No-GO" direction. In addition, the suture cannot travel through the locking aperture from the exit side to entrance side without complete suture failure. The purpose is to maximize the forces in the "No-Go" direction, while minimizing the forces in the "GO" direction to create as large a differential as possible. Maximizing this differential does two things: firstly, it allows the suture to easily pass through the locking aperture in the "GO" direction with minimal force which reduces or eliminates kinking or crimping of the suture. If greater forces were utilized in an attempt to repair the tissue, the force of pulling the suture through the locking aperture, might tear tissue or pull the implant away from the tissue, and secondly, maximizing the "NO-GO" forces allows the tissue restrain/capture to hold tissue with forces approaching the strength the suture itself. This disclosed technology confers at least a 1.5:1 "NO-GO":GO ratio and has approached ratios exceeding a 5:1 ratio. This design of the tissue restraint/suture capture allows these ratios to remain constant despite the size of the tissue restraint/suture capture. Sizes of 4.5mm, 7mm, 10mm and 20 mm in diameter are provided, which increases the "NO-GO" and 'Go" forces, however, the ratio between these various sizes typically remaining similar and not limited to these sizes alone.

It is therefore an object of this invention to provide a surgical suture system for tissue repair and reattachment of torn tissue together, to a tissue substrate or medical, veterinary or dental implant.

A broad aspect of this disclosure provides for the reattachment of any torn or damaged tissue or artificial tissue to any form of tissue substrate or together by the use of a uniquely configured tissue restraint/suture capture used alone or with other forms of tissue restraints, the tissue restraint/suture capture having a unique locking aperture arrangement for receiving a suture having spaced apart protuberances along the length of the suture. The tissue restrain/suture capture is configured for movement of the suture itself through the locking aperture in only one direction so that any lightening movement of the suture within the tissue restraint/suture capture is locked from reverse movement therebetween.

The following embodiments and aspects thereof are described and illustrated in conjunction with systems, tools and methods which are meant to be exemplary and illustrative and not limiting in scope. In various embodiments one or more of the above described problems have been reduced or eliminated while other embodiments are directed to other improvements. In addition to the exemplary aspects and embodiments described above, further aspects and embodiments will become apparent by reference to the drawings and by study of the following descriptions.

### BRIEF DESCRIPTION OF THE DRAWINGS

For a fuller understanding of the nature and objects of the invention, reference should be made to the following detailed description taken in connection with the accompanying drawings in which.
Figure 1 is a perspective view of a preferred embodiment of a tissue restraint/suture capture
Figure 2 is a perspective section view of the tissue restraint/suture capture of Figure 1.
Figure 3 is an elevation view of Figure 2 showing a beaded suture 40 secured within the tissue restraint/suture capture 10.
Figure 3A is an elevation view similar to Figure 3 showing a beaded suture 40 passing through the tissue restraint/suture capture 10.
Figure 3B is an elevation view similar to Figure 3 showing a beaded suture 40 resisting a force from the tissue restraint/suture capture 10.
Figure 4 is an elevation view of Figure 1.
Figure 5 is a top plan view of Figure 1.
Figure 5A is a top plan view similar to Figure 5 showing a beaded suture 40 passing through the tissue restraint/suture capture 10.
Figure 6 is a bottom plan view of Figure 1.
Figure 7 is a perspective section view of a suture anchor configured to receive the tissue restraint/suture capture 10 of Figure 1.
Figures 8 to 10 are side elevation section views of other embodiments of the suture anchor showing the tissue restraint/suture capture 10 of Figure 1 lockingly engaged with the beaded suture 40.
Figures 11 and 12 are top plan section views of other embodiments of the suture anchor showing alternate embodiments of the tissue restraint/suture capture of Figure 1 lockingly engaged therein.
Figures 13 and 14 are broken elevation section views of the enlarged ends of other embodiments of the suture member showing alternate embodiments of the tissue restraint/suture capture of Figure 1 lockingly engaged therein.
Figure 15 is a top plan view of another embodiment of the tissue restraint/suture capture of Figure 1.
Figure 16 is a perspective view of Figure 15.
Figure 17 is a section view in the direction of arrows 17-17 in Figure 15 showing the beaded suture 40.
Figure 18 is a top plan view of another embodiment of the tissue restraint/suture capture of Figure 1.
Figure 19 is a perspective view of Figure 18.
Figure 20 is a section view in the direction of arrows 17-17 in Figure 15 showing the beaded suture 40.
Figures 21 and 22 are side elevation section views of other embodiments of suture anchors showing the tissue restraint/suture capture 10 of Figure 1 lockingly engaged therein showing the beaded suture 40.
Figure 23 is a top plan view of Figure 22.
Figure 24 is a pictorial view of the tissue restraint/suture capture of Figure 1 lockingly engaged with a typical surgically implanted device showing the beaded suture 40.

Exemplary embodiments are illustrated in reference figures of the drawings. It is intended that the embodiments and figures disclosed herein are to he considered to be illustrative rather than limiting.

Similar reference characters refer to similar parts throughout the several Figures of the drawings.

### DETAILED DISCUSSION

### Nomenclature

- 10.: tissue restrain/suture capture
- 12.: body
- 14.: suture locking aperture
- 16.: radial slot
- 18.: slot relief hole
- 20.: flex arm slot
- 22.: flex arm
- 23.: flex arm pivot
- 24.: locking aperture inlet
- 26.: locking restriction aperture
- 28.: reverse restriction
- 30.: suture bead slot
- 32.: suture bead slot approach
- 40.: suture
- 42.: suture strand
- 44.: suture bead
- 50.: tissue anchor engaged with restraint/capture 10
- 52.: tissue anchor
- 54.: tapered spiral thread
- 56.: tissue penetrating tip
- 58.: head
- 60.: restraint receiving cavity
- 62.: suture exist passage
- 64.: suture entrance passage
- 70.: tissue anchor engaged with restraint/capture 10
- 72.: tissue anchor
- 74.: tapered spiral thread
- 76.: tissue penetrating tip
- 78: bead
- 80.: restraint receiving cavity
- 82.: suture clearance cavity
- 90.: tissue anchor engaged with restraint/capture 10
- 92.: tissue anchor
- 94.: tapered spiral thread
- 96.: tissue penetrating tip
- 98.: head
- 100.: suture passage
- 102.: restraint receiving cavity
- 104.: indent
- 105.: annular groove
- 106.: imple
- 107.: annular bead
- 110, 110': tissue restraint/suture capture
- 112,: body
- 114.: suture locking aperture
- 116.: radial slot
- 118.: slot relief hole
- 120.: flex arm relief hole
- 122.: flex arm
- 124, 124'.: suture clearance notch
- 126.: locking aperture restriction
- 130, 130'.: tissue anchor engaged with restraint/capture 10
- 132, 132'.: tissue anchor
- 134, 134': tapered spiral thread
- 136.: tissue penetrating tip
- 138, 138': head
- 140, 140': restraint receiving cavity
- 142, 142'.: suture clearance cavity
- 150.: tissue anchor engaged with restraint/capture 10
- 152.: tissue anchor
- 154.: tapered spiral thread
- 156.: tissue penetrating tip
- 158.: head
- 160.: restraint receiving cavity
- 162.: suture clearance cavity
- 164.: bead clearance
- 166.: bead exit
- 170.: tissue anchor engaged with dual restraints/captures 10
- 172.: tissue anchor
- 174.: tapered spiral thread
- 176.: tissue penetrating tip
- 178.: head
- 180.: restraint receiving cavity
- 182.: restraint receiving cavity
- 184.: suture passage
- 190.: prosthesis implant
- 192.: restraint receiving cavity

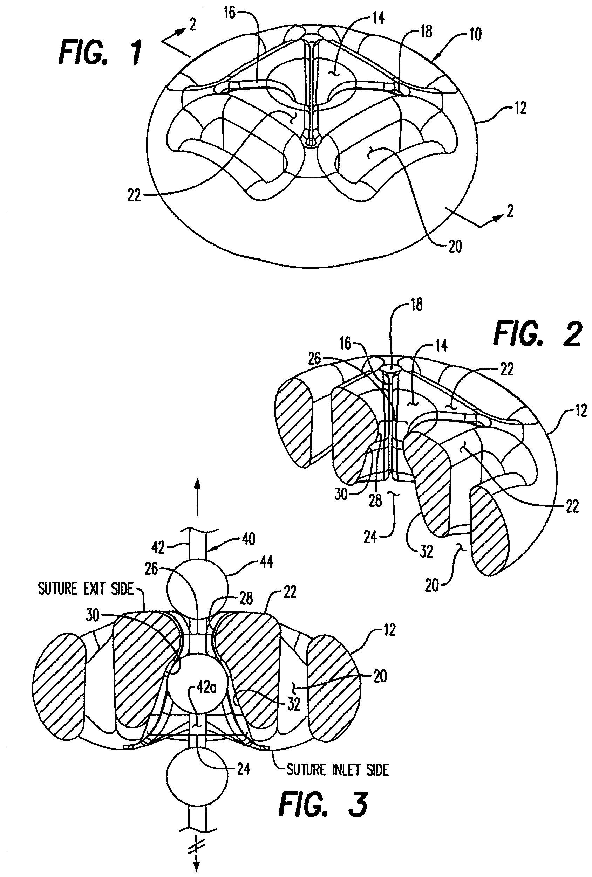

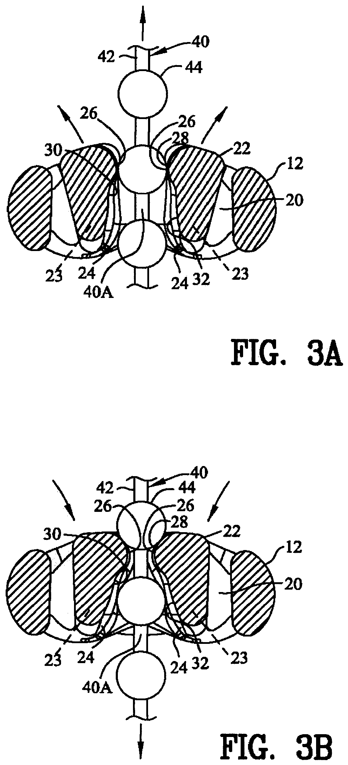

Referring now to the drawings, and firstly to Figures 1 to 6, the preferred embodiment of a tissue restraint/suture capture is there shown generally at numeral 10. This tissue restraint/suture capture 10, sometimes referred to herein as a "restraint/capture", is preferably formed of flexible or semi-flexible medically implantable material, such as high density polyethylene, although other suitable material may be utilized. This tissue restraint/suture capture 10 has been shown through computer mathematical analysis to provide for at least preferably a 5: 1 ratio of force to move a beaded suture 40 (the suture 40 having spherical or bead-shaped protuberances 44 longitudinally spaced apart along a slender strand 42 and being formed of a flexible, or semi-flexible medically implantable material) through the suture locking aperture 14 of the body 12 in the direction of the arrow shown in Figure 3 to approximately four times more force to move the suture backward through the locking aperture 14 in the opposite direction. This provides the surgeon performing reattachment surgery the confidence that this tissue restraint/suture capture 10 will maintain the applied force to reconnect adjacent or mating tissues as applied by the surgeon.

Details of this tissue restraint/suture capture 10 were developed initially using Finite Element Analysis (FEA) and its proof-of-concept was bench tested with an Instron, supporting the preliminary FEA findings. The engineering was performed and perfected so failure of the suture/capture system was the suture itself and not the tissue restraint/suture capture. This design 10 also minimizes "GO" force, and maximize "NO-GO" force, the locking aperture 14 specifically engineered to maximize this effect and approaches suture ultimate tensile strength, while the 'GO" forces approximate a simple hand pull. This "NO-GO"/"GO" differential, by minimizing "GO" and maximizing "NO-GO", allows the surgeon to work with confidence with the knowledge that there is no need to pull too hard to get the suture to advance, which could tear the tissue or pull-out the repair, while knowing that the tissue restraint/suture capture 10 can hold the tissue in place with forces approaching the failure strength of the suture.

This tissue restraint/suture capture 10 includes the central suture locking aperture 14 which extends axially and orthogonally to an imaginary plane of the flattened oval shape body 12. The tissue restraint/suture capture 10 is a one-piece unit with a height of the flattened oval shape body 12 being less than one-half of the diameter of the flattened oval shape body 12. It should be appreciated by those skilled in the art that the outer diameter of the flattened oval shape body 12 may be expanded further to add additional strength when implanted into tissue.

The tissue restraint/suture capture 10 defines a locking aperture inlet 24 substantially larger than the diameter of the suture beads 44 for ease of insertion of the tissue restraint/suture capture 10 thereinto. The depth of the inlet side of this suture locking aperture 14 is such that, as best seen in Figure 3, a portion of the suture strand 42 may be easily grasped during a surgical procedure to begin to pull the suture 40 in the direction of the arrow through the suture locking aperture 14. The locking aperture inlet 24 allows the suture 40 with its beads 44 to approach from various angles thereby allowing more degrees of freedom for the suture 40 to enter the locking aperture 14 without binding, crimping, or bending the suture strand 42.

As each of the suture beads 44 is guided by slot approach 32 and bears against the suture head seat 30, applying further pressure axially to the suture 40 in the direction of the arrow causes the mating inner contour of each of the flex arms 22 (as described more fully herebelow) to flex outwardly (upwardly as viewed in Figure 3) to expand the locking aperture restriction 26 until the desired number of suture bead 44 are pulled therethrough, after which the flex arms 22 automatically and resiliency return to the at rest position, collapsing the locking aperture restriction 26. Importantly, the outlet end of the suture locking aperture 14 includes a reverse restriction 28 which serves to insure that the suture 40 may not be moved in the opposite ("NO-GO") direction from the arrow without applying a pulling force typically necessary to fracture the suture strand 42, the flex arms 22 resiliency collapsing inwardly to narrow the locking restriction aperture 26.

Each of the flex arms 22 is defined by adjacent radially extending radial slots 16 each of which terminate at the outer end thereof in an enlarged slot relief hole 18. Each of the flex arms 22 is further defined by an elongated oval or racetrack-shaped elongated flex arm slot 20. Each inner corner of each of the flex arm slots 20 is formed in proximity to one of the slot relief holes 18, the spacing therebetween having a substantial influence on the overall flexibility of each of the Hex arms 22 as the suture 40 and each of its suture beads 44 is pulled through the suture locking aperture 14 as previously described.

Figure 3A is an elevation view similar to Figure 3 showing a beaded suture 40 passing through the tissue restraint/suture capture 10 as shown by the arrow. Each of the flex arms 22 flexes upwardly and outwardly about a flex arm pivot 23 as shown in Figure 3A to expand the locking aperture restriction 26.

Figure 3B is an elevation view similar to Figure 3 showing a beaded suture 40 resisting a reverse force from the tissue restraint/suture capture 10 as shown by the arrow. Each of the flex arms 22 flexes downwardly and inwardly about a flex arm pivot 23 as shown in Figure 3B to contract the locking aperture restriction 26.

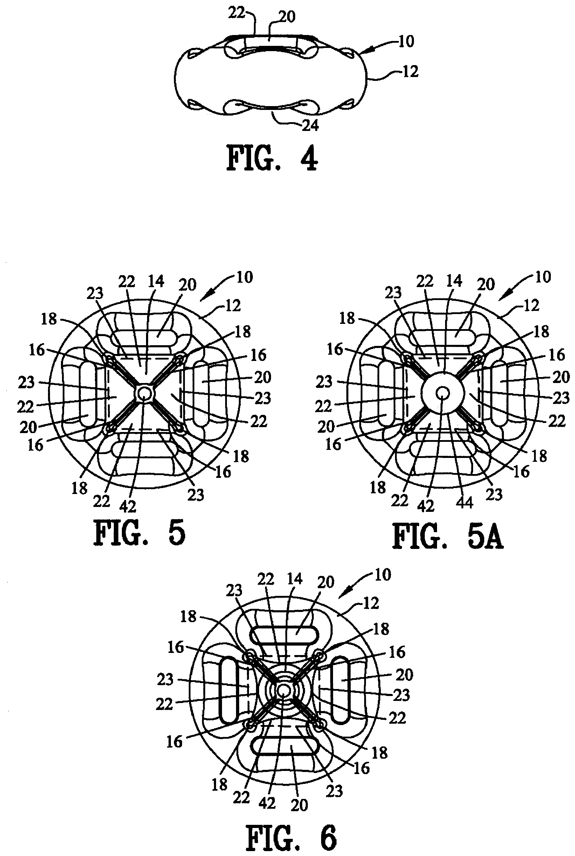

Figure 5 is a top plan view of Figure 1 showing the suture 40 passing through the tissue restraint/suture capture 10. The suture 40 passes freely through the locking aperture restriction 26.

Figure 5A is a top plan view similar to Figure 5 showing the suture bead 44 of the beaded suture 40 passing through the tissue restraint/suture capture 10. Each of the flex arms 22 flexes about the flex arm pivot 23 as shown in Figure 5A to expand the locking aperture restriction 26.

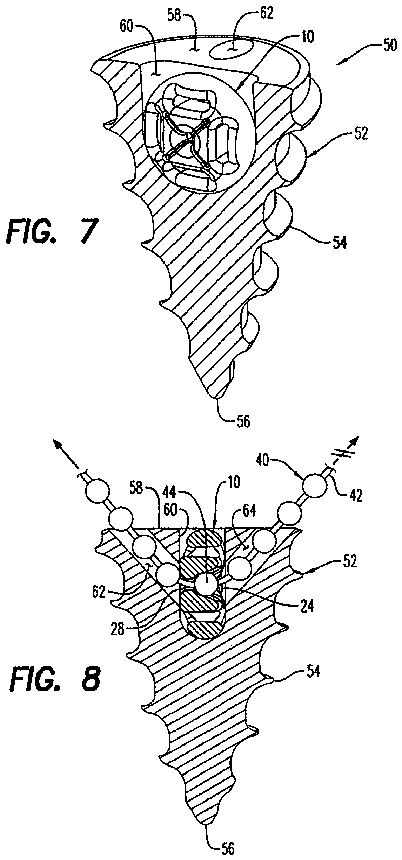

Referring now to Figures 7 and 8. a first embodiment of the combination of the previously described tissue restraint/suture capture 10 and a tissue anchor 52 is there shown generally at numeral 50. The tissue anchor 52, preferably formed of surgically implantable material suitably formed to he rotatably driven into cartilage or bone, includes a tapered spiral thread 54 and a tissue penetrating tip 56 for ease of piercing through the tissue material as the tissue anchor 52 is rotatably driven into and secured within the cartilage or bone material.

The head 58 of the tissue anchor 52 includes a longitudinally extending restraint receiving cavity 60 formed to tightly and wedgingly receive the tissue restraint/suture capture 10 into the position best seen in Figure 8. Diagonally extending suture entrance and exit passages 64 and 62 are provided so that the suture 40 may be easily inserted through the suture locking aperture 14 of the tissue restraint/suture capture 10 through the suture entrance passage 64, exiting from the suture exit passage 62. After the tissue anchor 52 has been secured within the tissue by being rotationally driven as facilitated by the openings of each of the passages 62 and 64, the suture 40 may be inserted into the position shown in Figure 8 and then tensioned as desired. Again, by the structural benefits of the tissue restraint/suture capture 10 as previously described, movement of the suture 40 is only possible, preferably without suture fracture, in the direction of the arrow from the suture exit passage 62.

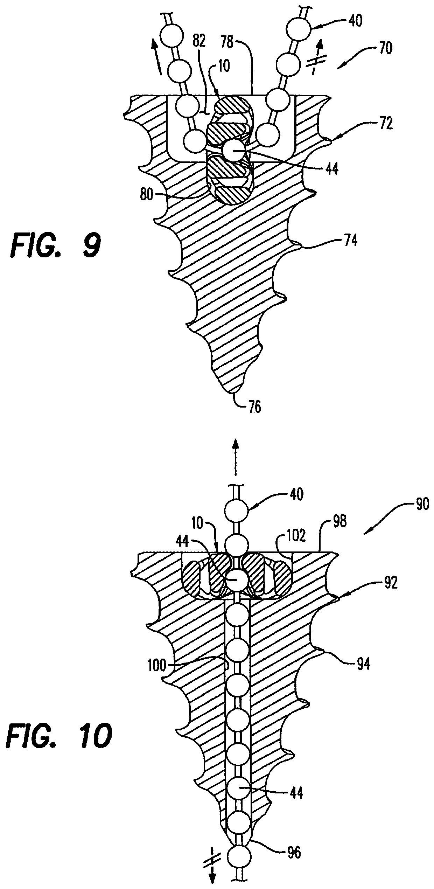

Referring now to Figure 9, another embodiment of the combination of the suture tissue restraint/suture capture 10 and a tissue anchor 72 is there shown generally at numeral 70. In this embodiment 70, the tissue anchor 72, having tapering spiral threads 74 and a tissue penetrating tip 76 as previously described, includes a restraint receiving cavity 80 which tightly and wedgingly receives a portion of the tissue restraint/suture capture 10 in an upright orientation. However, an enlarged suture clearance cavity 82 formed axially downwardly into the head 78 is provided for free access of the suture 40 into and from the suture locking aperture 14.

In Figure 10, yet another embodiment of the combination of a tissue anchor 92 and the tissue restraint/suture capture 10 is there shown generally at numeral 90. In this embodiment 90, the tissue restraint/suture capture 10 is oriented transversely to the longitudinal axis of the tissue anchor 92, the tissue restraint/suture capture 10 being tightly and wedgingly secured into a generally circular restraint receiving cavity 102 formed into the head 98 of the tissue anchor 92. To facilitate smooth and easy access of the suture 40 through the suture locking aperture 14, an elongated longitudinally extending suture passage 100 is provided. In this embodiment 90, the tissue anchor 92 may be secured into the tissue after the suture 40 has been fed from the opposite side of the anchoring tissue and then fed through the suture passage 100 and the suture locking aperture 14 of the tissue anchor 92 for tensioning in the direction of the arrow. Note that the force applied downwardly on the tissue restraint/suture capture 10 after proper tensioning of the suture 40 will enhance maintaining the tissue restraint/suture capture 10 within the suture receiving cavity 102.

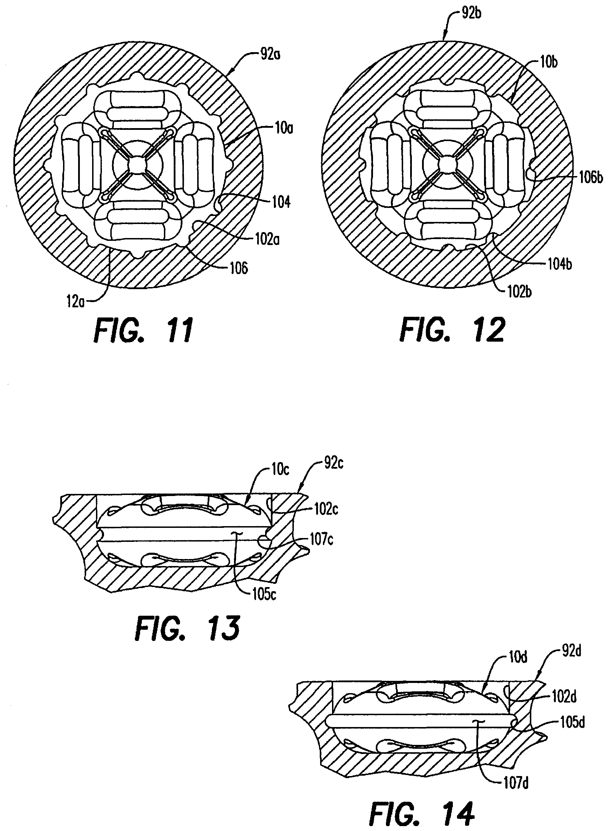

Referring now to Figures 11 to 14, rather than relying only upon the tight, frictional wedging retention of the tissue restraint/suture capture 10a, b, e, d within the cavity formed into the head of a tissue anchor, four separate additional structural features are shown in Figures 11 to 14. In Figure 1.1, the head of the tissue anchor 92a includes indents 104 which securely engage with spaced dimples 106 which outwardly extend from the perimeter of the body 12a of the tissue restraint/suture capture 10a. In Figure 12, the reverse structural feature is provided wherein dimples 106b extend inwardly front the restraint receiving cavity 102b to engage into indents 104b formed into the body of the tissue restraint/suture capture 10b. In Figure 13, an annular groove 105c is formed into the periphery of the body of tissue restraint/suture capture 10ce which snappingly engages with an annular bead 107c formed inwardly from the restraint receiving cavity 102c. In Figure 14, the reverse structure having the annular bead 107d formed outwardly extending from the body of the tissue restraint/suture capture 10d matingly engages into the annular groove 105d formed into the restraint receiving cavity 102d. Other interfering structure to effect this locking engagement is also anticipated within the scope of this disclosure.

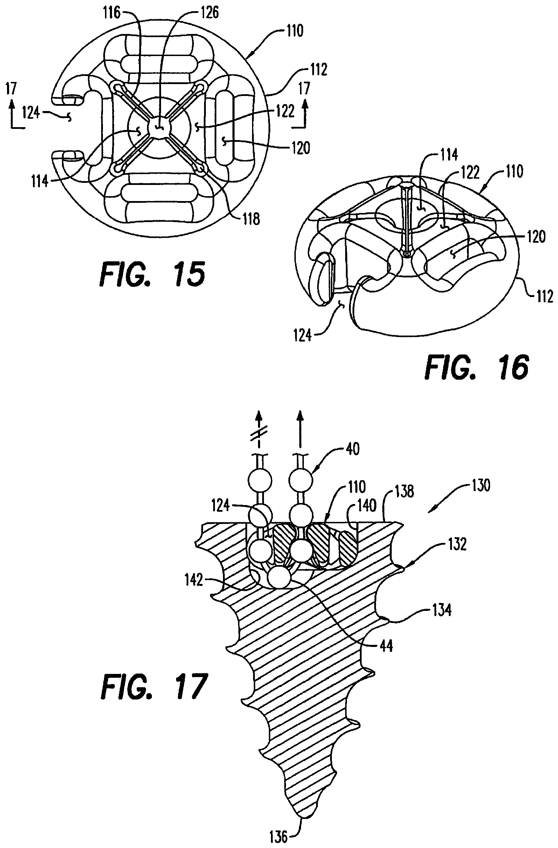

Alternate forms of suture clearances are shown in Figures 15 to 20 in conjunction with uniquely configured heads 138 and 138' of tissue anchors 132 and 132'. In Figures 15 and 16, a suture clearance notch 124 is formed into the periphery of the body 112 directly into one of the flex arm relief holes 120, This clearance notch 124 is sufficiently wide for the suture 40 to pass thereinto when the tissue restraint/suture capture 110 is secured into the restraint receiving cavity 140 formed into the head 138 of the tissue anchor 132. An additional suture clearance cavity 142 is provided so that the suture 40 may be reversed as it is inserted into the locking aperture of the tissue restraint/suture capture 110. This tissue anchor 132 as previously described, also includes the tapered spiral threads 134 and the tissue penetrating tip 136.

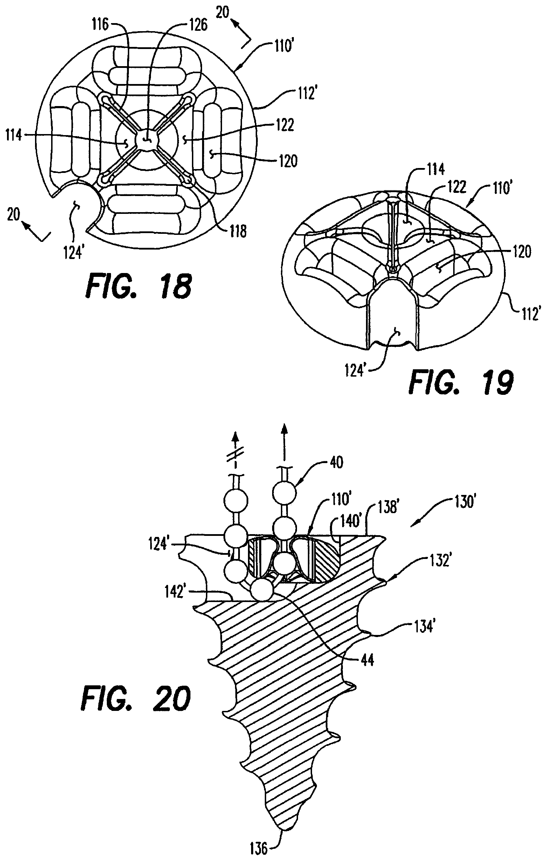

In Figures 18 to 20, the combination of the tissue restraint suture capture 110' and the tissue anchor 132' is shown in combination at 130'. The tissue restraint/suture capture 110' is structurally generally as previously described except for having the suture clearance notch 124' formed into the periphery of the body 112' directly radially outwardly from one of the slot relief holes 118 disposed at the radially outward end of the corresponding radial slot 116. Again, the suture clearance notch 124' has sufficient width and depth so that the suture 40 will pass thereinto with additional clearance by suture clearance cavity 142' to reverse the suture direction to pass into the tissue restraint/suture capture 110' which has been secured into the restraint receiving cavity 140' formed into the head 138' of the tissue anchor 132'.

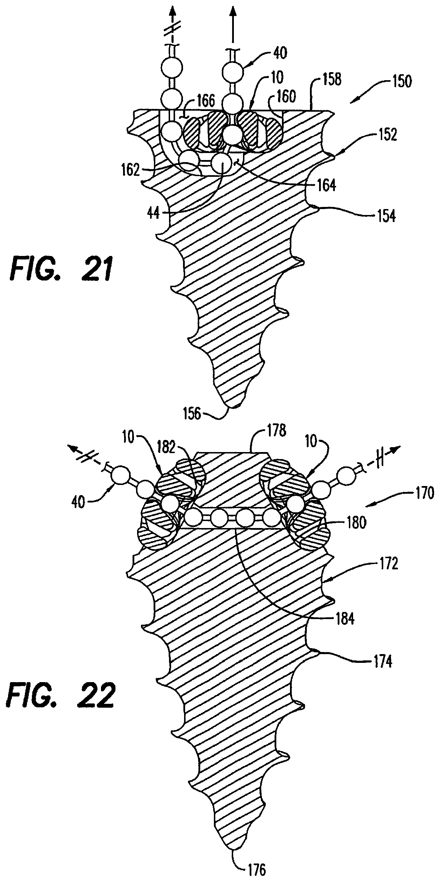

Still another arrangement for the locking interengagement between the suture tissue restraint/suture capture 10 and a tissue anchor 152 is there shown generally at numeral 150 in Figure 21. In this embodiment 150, the tissue restraint/suture capture 10 is lockingly engaged into a mating restraint receiving cavity 160 and a suture clearance cavity 162 having a bead clearance area at 164 and a bead exit area 166 is also provided for the smooth introduction of the suture 40 first downwardly into the bead exit area 166 and then upwardly from the locking aperture of the tissue restraint/suture capture 10. The tissue anchor 152 similarly includes tapered spiral thread 1.54 and a tissue penetrating tip 156 as previously described.

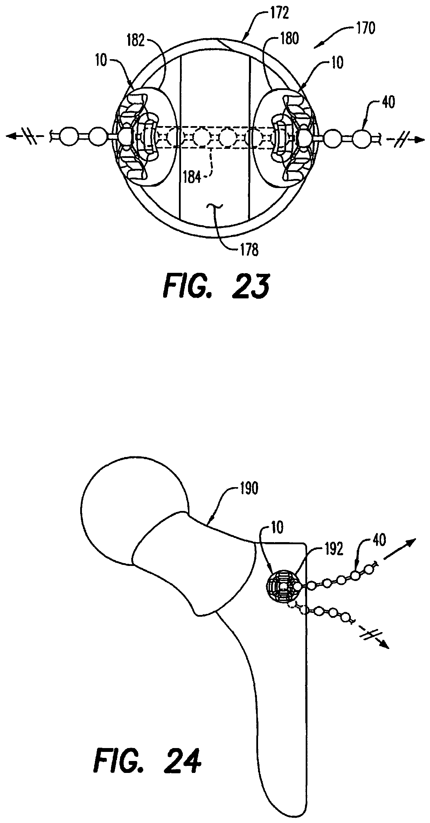

In Figures 22 and 23, a tissue anchor 172 with dual opposing restraints/captures 10 is shown generally at numeral 170. In this embodiment 170, opposing tissue restraints/suture captures 10 are secured within diagonally oriented opposing restraint receiving cavities 180 and 182 such that the suture 40 may be lockingly passed through one of the tissue restraints/suture captures 10, then through a connecting suture passage 184 and then into an through the opposing tissue restrain/suture capture 10. Note that, in this embodiment 170, the suture 40 is restrained from any movement whatsoever with respect to the head 178 of the tissue anchor 172, requiring that the suture 40 be first passed through one of the tissue restraints/suture captures 10 then through suture passage 184 and then tensioned through the second tissue restrain/suture capture 10 after it has been positioned in its restraint receiving cavity 182. Alternately, each tissue restrain/suture capture 10 (in this embodiment 170, a suture capture) may be slid down the suture 40 on each side of the tissue anchor 172 and locked in place as shown rather than having the suture 40 pass through the suture 40 pass through the suture passage 18 with the tissue restraint/suture captures 10 already in place. Another arrangement envisions reversing one of restrain/suture captures 10, allowing the suture 40 to travel in only one direction through one tissue restrain/suture capture 10, then through the suture passage 184 and out through the second tissue restrain/suture capture 10 for enhanced "NO-GO" strength.

Lastly, in Figure 24, the tissue restrain/suture capture 10 is shown secured into a mating restraint receiving cavity 192 formed into a prosthetic implant 192. This concept is intended to shown that the tissue restraint/suture capture 10 or any suitable variation thereof within the scope of this invention may be secured into any medically, veterinary, dental implantable prosthesis implant such as a pacemaker, heart valve, or stent, veterinary or medical dental bone plate, breast implant, gastrointestinal ligature or ligature type device or any other medically, veterinary, dental implantable device which either requires suture restraint or will provide a suture restraint for a suture to pass therethrough. Thus, the tissue restraint/suture capture 10, as opposed to being utilized as a tissue restraint alone or with an anchor, may be used as a suture capture incorporated into other medical, veterinary or dental implants.

While a number of exemplary aspects and embodiments have been discussed above, those of skill in the art will recognize certain modifications, permeations and additions and subcombinations thereof are within the level of those skilled in the art. It is therefore intended that the following appended claims and claims hereinafter introduced are interpreted to include all such modifications, permeations, additions and subcombinations that are within their true scope.

Although the invention has been described in its preferred form with a certain degree of particularity, it is understood that the present disclosure of the preferred form has been made only by way of example.

## Claims

1. Surgical suture system for tissue repair and reattachment of torn tissue to a tissue substrate or medical, veterinary or dental implant, the system comprising an elongated flexible suture (40) having a plurality of longitudinally spaced protuberances (44) along a length thereof
a tissue restraint/suture capture (10) having a central locking aperture (14) for receiving a suture member of the suture (40) passed therethrough for longitudinal movement of the suture (40) in only a forwardly direction through the locking aperture (14), and
a plurality of flex arms (22) defined by adjacent radially extending radial slots (16), **characterized in**
**that** each of the radial slots (16) terminates at the outer end thereof in a slot relief hole (18) and
**that** each of the flex arms (22) is further defined by an elongated flex arm slot (20) and
**that** each inner corner of the flex arm slots (20) is formed in proximity to one of the slot relief holes (18), such that the spacing between the relief holes (18) and the inner corners substantially influences
the overall flexibility of the flex arm (20).

2. Surgical suture system of Claim 1, wherein the system comprises an implant, the implant including an implantable body being configured to securely receive the tissue restraint/suture capture (10).

3. Surgical suture system of Claim 1 or 2, wherein a locking aperture inlet (24) of the central locking aperture (14) is substantially larger than each of the protuberances (44) along the flexible suture (40) for enabling insertion from various angles of the flexible suture (40) into the central locking aperture (14),

4. Surgical suture system of any of the preceding Claims, further comprising a tissue anchor or prosthesis implant (190) having a cavity for receiving and securing said tissue restraint/suture capture (10) therein, wherein the suture (40) is securable to the tissue anchor or prosthesis implant (190).

5. Surgical suture system of any of the preceding Claims, wherein said tissue restraint/suture capture (10) further comprises:
a plurality of spaced apart radial slots (16) each radially extending outwardly from said locking aperture (14) toward but not to a periphery of a flattened oval-sectioned body (12) of the tissue restraint/suture capture (10) to define a flex arm (22) between each of adjacent the radial slots (16); and a radially innermost end of each of said flex arms (22) defining a locking restriction aperture (26) of the locking aperture (14).

6. Surgical suture system of Claim 5, wherein said tissue restraint/suture capture (10) further comprises:
a flex arm slot (20) formed through a mid portion of each of the flex arms (22) and being positioned in evenly spaced relation between a closed end of each of adjacent the radial slots (16); and/or
the flex arm slots (20) for controlling the force required to pull the suture (40) through the locking aperture (14); and/or
the locking restriction aperture (26), when the flex arms (22) are relaxed, being substantially smaller in diameter than a diameter of the protuberances (44); and/or
the locking aperture (14) having an enlarged locking aperture inlet (24) adjacent a suture inlet side of the body (12) for receiving the suture (40) and to allow the suture (40) to pass therethrough and be urged through the locking restriction aperture (26); and/or
the locking aperture (14) having a reverse restriction adjacent a suture exit side of said body (12) for restricting the suture (40), once having been pulled through the locking aperture (14), from being moved in a reverse direction of movement.

7. Surgical suture system of any of the preceding claims, wherein the surgical tissue restraint/suture capture (10) comprising a body including a locking aperture (14) sized to receive an elongated slender flexible suture (40) having a plurality of longitudinally spaced protuberances (44) along a length thereof passing through the locking aperture (14) to cooperatively restrict longitudinal movement of the suture (40) to only a forwardly direction of movement through the locking aperture (14).

8. Surgical suture system of Claim 7, further comprising a tissue anchor, a prosthesis, or an implantable device serving another purpose than as an anchor having a cavity for receiving and securing said tissue restraint/suture capture (10) therein, wherein the suture (40) is securable to the tissue anchor or prosthesis implant (190).

9. Surgical suture system of Claim 7 or 8, the tissue restraint/suture capture (10) further comprising a plurality of spaced apart radial slots (16) each radially extending outwardly from said locking aperture (14) toward but not to a periphery of a flattened oval-sectioned body to define a flex arm (22) between each of adjacent the radial slots (16); and a radially innermost end of each of said flex arms (22) defining a locking restriction aperture (26) of the locking aperture (14).

10. Surgical suture system of Claim 9, further comprising a flex arm slot (20) formed through a mid portion of each of the flex arms (22) and being positioned in evenly spaced relation between a closed end of each of adjacent the radial slots (16); preferably the flex arm slots (20) for controlling the force required to pull the suture (40) through the locking aperture (14); preferably
the locking restriction aperture (26), when the flex arms (22) are relaxed, being substantially smaller in diameter than a diameter of the protuberances (44); and/or
the locking aperture (14) having an enlarged locking aperture inlet adjacent a suture inlet side of the body (12) for receiving the suture (40) and to allow the suture (40) to pass therethrough and be urged through the locking restriction aperture (26); and/or
the locking aperture (14) also having a reverse restriction adjacent a suture exit side of said body (12) for restricting the suture (40), once having been pulled through the locking aperture (14), from being moved in a reverse direction of movement by reducing the size of the locking restriction aperture (26) as the flex arms (22) are flexed in the reverse direction of force applied to the suture (40).

11. Surgical suture system of any of the preceding Claims, wherein the locking aperture (14) permits entrance of the suture (40) thereinto with minimal force and maximizing force that preventing the suture (40) from moving in the reverse direction, creating a differential force ratio between the force required to move the suture in the reverse direction, referred to as a "NO-GO" force, and the force required to move the suture in the forward direction, referred to as a "GO" force, exceeding a 1.5:1 ratio.

12. Surgical suture system of any of the preceding Claims, wherein each of the protuberances (44) is guided by a slot approach (32) and bears against the suture head seat, such that applying further pressure axially to the suture (40) causes the mating inner contour of each of the flex arms (22) to flex outwardly to expand the locking aperture restriction until the desired number of protuberances (44) are pulled therethrough, preferably after which the flex arms (22) automatically and resiliently return to the at rest position, collapsing the locking aperture restriction.

13. Surgical suture system of any of the preceding Claims, wherein the outlet end of the suture locking aperture (14) includes a reverse restriction which serves to insure that the suture (40) may not be moved in the opposite direction, in particular referred to as "NO-GO" direction, without applying a pulling force typically necessary to fracture a suture strand (42), the flex arms (22) resiliently collapsing inwardly to narrow the locking restriction aperture (26).

14. Surgical suture system of any of the preceding Claims, wherein the flex arm slots are elongated oval or racetrack-shaped elongated.

15. Surgical suture system of any of the preceding Claims, wherein the locking aperture (14) of the tissue restrain/suture capture (10) provides for higher resistance on the exit side of the locking aperture (14) and lower resistance on the entrance side of the locking aperture (14), preferably:
such that the suture (40) easily passes through the entrance to the locking aperture (14) of the tissue restrain/suture capture (10) and leaves through the exit side of the locking aperture (14), both allowing the suture (40) to travel forward, referred to as "Go"-direction; and/or
such that preventing the suture (40) from travelling in reverse or pulled backward from the entrance to the locking aperture (14), referred to as "No-Go" direction,
in particular:
such that the suture (40) cannot travel through the locking aperture (14) from the exit side to entrance side without complete suture failure; and/or
such that the forces in the "No-Go" direction are maximized, while minimizing the forces in the forward direction to create a differential.

## Patentansprüche

1. Chirurgisches Nahtmaterialsystem zur Gewebereparatur und Wiederanheftung von gerissenem Gewebe an ein Gewebesubstrat oder medizinisches, veterinärmedizinisches oder Zahnimplantat, das System aufweisend ein längliches flexibles Nahtmaterial (40), das mehrere in Längsrichtung beabstandete Verdickungen (44) entlang einer Länge davon aufweist,
eine Geweberückhaltung / einen Nahtmaterialfang (10) mit einer zentralen Verriegelungsöffnung (14) zur Aufnahme eines Nahtmaterialelements des Nahtmaterials (40), das dort hindurchgeführt ist, zur Längsbewegung des Nahtmaterials (40) nur in einer Vorwärtsrichtung durch die Verriegelungsöffnung (14), und
mehrere Flexarme (22), die durch benachbarte sich radial erstreckende radiale Schlitze (16) definiert sind,
**dadurch gekennzeichnet,**
**dass** jeder der radialen Schlitze (16) an seinem äußeren Ende in einem Schlitzentlastungsloch (18) endet und
**dass** jeder der Flexarme (22) ferner durch einen länglichen Flexarmschlitz (20) definiert ist und
**dass** jede innere Ecke der Flexarmschlitze (20) in der Nähe eines der Schlitzentlastungslöcher (18) ausgebildet ist, so dass der Abstand zwischen den Entlastungslöchern (18) und den inneren Ecken die Gesamtflexibilität des Flexarms (20) wesentlich beeinflusst.

2. Chirurgisches Nahtmaterialsystem nach Anspruch 1, wobei das System ein Implantat umfasst, wobei das Implantat einen implantierbaren Körper umfasst, der dazu abgebildet ist, die Geweberückhaltung / den Nahtmaterialfang (10) sicher aufzunehmen.

3. Chirurgisches Nahtmaterialsystem nach Anspruch 1 oder 2, wobei ein Verriegelungsöffnungseinlass (24) der zentralen Verriegelungsöffnung (14) wesentlich größer ist als jede der Verdickungen (44) entlang des flexiblen Nahtmaterials (40), um das Einführen aus verschiedenen Winkeln des flexiblen Nahtmaterials (40) in die zentrale Verriegelungsöffnung (14) zu ermöglichen.

4. Chirurgisches Nahtmaterialsystem nach einem der voranstehenden Ansprüche, ferner umfassend einen Gewebeanker oder ein Prothesenimplantat (190) mit einem Hohlraum zum Aufnehmen und Befestigen der Geweberückhaltung / des Nahtmaterialfangs (10) darin, wobei das Nahtmaterial (40) an dem Gewebeanker oder Prothesenimplantat (190) befestigbar ist.

5. Chirurgisches Nahtmaterialsystem nach einem der voranstehenden Ansprüche, wobei die Geweberückhaltung / der Nahtmaterialfang (10) ferner umfasst:
mehrere voneinander beabstandete radiale Schlitze (16), die sich jeweils von der Verriegelungsöffnung (14) radial nach außen in Richtung, jedoch nicht bis zu einem Umfang eines abgeflachten, oval geschnittenen Körpers (12) der Geweberückhaltung / des Nahtmaterialfangs (10) erstrecken, um einen Flexarm (22) zwischen jedem der benachbarten radialen Schlitze (16) zu definieren; und ein radial innerstes Ende von jedem der Flexarme (22), das eine Verriegelungsverengungsöffnung (26) der Verriegelungsöffnung (14) definiert.

6. Chirurgisches Nahtmaterialsystem nach Anspruch 5, wobei die Geweberückhaltung / der Nahtmaterialfang (10) ferner umfasst:
einen Flexarmschlitz (20), der durch einen mittleren Abschnitt jedes der Flexarme (22) gebildet ist und in einer gleichmäßig beabstandeten Beziehung zwischen einem geschlossenen Ende von jedem der benachbarten radialen Schlitze (16) positioniert ist; und/oder
die Flexarmschlitze (20) zum Kontrollieren der Kraft, die erforderlich ist, um das Nahtmaterial (40) durch die Verriegelungsöffnung (14) zu ziehen; und/oder
wobei die Verriegelungsverengungsöffnung (26), wenn die Flexarme (22) entspannt sind, im Durchmesser wesentlich kleiner als ein Durchmesser der Verdickungen (44) ist; und/oder
wobei die Verriegelungsöffnung (14) einen vergrößerten Verriegelungsöffnungseinlass (24) angrenzend an eine Nahtmaterialeinlassseite des Körpers (12) aufweist, um das Nahtmaterial (40) aufzunehmen und damit das Nahtmaterial (40) dort hindurch gelassen und durch die Verriegelungsverengungsöffnung (26) gedrückt werden kann; und/oder
wobei die Verriegelungsöffnung (14) eine Rückwärtsbeschränkung angrenzend an eine Nahtaustrittsseite des Körpers (12) aufweist, um zu verhindern, dass das Nahtmaterial (40) nach dem Ziehen durch die Verriegelungsöffnung (14) in eine umgekehrte Bewegungsrichtung bewegt wird.

7. Chirurgisches Nahtmaterialsystem nach einem der voranstehenden Ansprüche, wobei die chirurgische Geweberückhaltung / der Nahtmaterialfang (10) einen Körper mit einer Verriegelungsöffnung (14) umfasst, die zur Aufnahme eines länglichen, schlanken, flexiblen Nahtmaterials (40), das mehrere in Längsrichtung beabstandete Verdickungen (44) entlang seiner Länge aufweist, bemessen ist, die durch die Verriegelungsöffnung (14) verlaufen, um gemeinsam eine Längsbewegung des Nahtmaterials (40) auf nur eine Vorwärtsrichtung der Bewegung durch die Verriegelungsöffnung (14) zu begrenzen.

8. Chirurgisches Nahtmaterialsystem nach Anspruch 7, ferner umfassend einen Gewebeanker, eine Prothese oder eine implantierbare Vorrichtung, die einem anderen Zweck dient als ein Anker mit einem Hohlraum zum Aufnehmen und Befestigen der Geweberückhaltung / des Nahtmaterialfangs (10) darin, wobei das Nahtmaterial (40) an dem Gewebeanker oder Prothesenimplantat (190) befestigbar ist.

9. Chirurgisches Nahtmaterialsystem nach Anspruch 7 oder 8, wobei die Geweberückhaltung / der Nahtmaterialfang (10) ferner mehrere voneinander beabstandete radiale Schlitze (16) aufweist, die sich jeweils radial von der Verriegelungsöffnung (14) nach außen in Richtung, aber nicht bis zu einem Umfang eines abgeflachten oval geschnittenen Körpers erstrecken, um einen Flexarm (22) zwischen jedem der benachbarten radialen Schlitze (16) zu definieren; und ein radial innerstes Ende jedes der Flexarme (22), die eine Verriegelungsverengungsöffnung (26) der Verriegelungsöffnung (14) definieren.

10. Chirurgisches Nahtmaterialsystem nach Anspruch 9, ferner umfassend einen Flexarmschlitz (20), der durch einen mittleren Abschnitt jedes der Flexarme (22) gebildet ist und in gleichmäßig beabstandeter Beziehung zwischen einem geschlossenen Ende von jedem der benachbarten radialen Schlitze (16) positioniert ist; vorzugsweise
die Flexarmschlitze (20) zum Kontrollieren der Kraft, die erforderlich ist, um das Nahtmaterial (40) durch die Verriegelungsöffnung (14) zu ziehen; vorzugsweise
wobei die Verriegelungsverengungsöffnung (26), wenn die Flexarme (22) entspannt sind, im Durchmesser wesentlich kleiner als ein Durchmesser der Verdickungen (44) ist; und/oder
wobei die Verriegelungsöffnung (14) einen vergrößerten Verriegelungsöffnungseinlass benachbart zu einer Nahtmaterialeinlassseite des Körpers (12) zum Aufnehmen des Nahtmaterials (40) und damit das Nahtmaterial (40) dort hindurch geführt und durch die Verriegelungsverengungsöffnung (26) gedrückt werden kann; und/oder
wobei die Verriegelungsöffnung (14) auch eine Rückwärtsbeschränkung angrenzend an eine Nahtausgangsseite des Körpers (12) aufweist, um zu verhindern, dass das Nahtmaterial (40), nachdem es durch die Verriegelungsöffnung (14) gezogen wurde, in einer umgekehrten Bewegungsrichtung bewegt wird, indem die Größe der Verriegelungsverengungsöffnung (26) verringert wird, da die Flexarme (22) in umgekehrter Richtung zu der auf das Nahtmaterial (40) ausgeübten Kraft gebogen werden.

11. Chirurgisches Nahtmaterialsystem nach einem der voranstehenden Ansprüche, wobei die Verriegelungsöffnung (14) den Eintritt des Nahtmaterials (40) darin mit minimaler Kraft zulässt und die Kraft, die verhindert, dass sich das Nahtmaterial (40) in der umgekehrten Richtung bewegt, maximiert, wodurch ein Differenzkraftverhältnis erzeugt wird zwischen der Kraft, die erforderlich ist, um das Nahtmaterial in die umgekehrte Richtung zu bewegen, die als "NO-GO"-Kraft bezeichnet wird, und der Kraft, die erforderlich ist, um das Nahtmaterial in Vorwärtsrichtung zu bewegen, die als "GO"-Kraft bezeichnet wird, das ein Verhältnis von 1,5:1 übersteigt.

12. Chirurgisches Nahtmaterialsystem nach einem der voranstehenden Ansprüche, wobei jede der Verdickungen (44) durch einen Schlitzansatz (32) geführt ist und an dem Nahtmaterial-Kopfsitz anliegt, sodass das Ausüben von weiterem Druck axial zu dem Nahtmaterial (40) bewirkt, dass sich die passende Innenkontur jedes der Flexarme (22), nach außen biegt, um die Verriegelungsöffnungsverengung aufzuweiten, bis die gewünschte Anzahl von Verdickungen (44) hindurchgezogen ist, vorzugsweise wobei danach die Flexarme (22) automatisch und elastisch in die Ruhestellung zurückkehren, wodurch die Verriegelungsöffnungsverengung zusammenklappt.

13. Chirurgisches Nahtmaterialsystem nach einem der voranstehenden Ansprüche, wobei das Austrittsende der Nahtmaterialverriegelungsöffnung (14) eine Rückwärtsbeschränkung aufweist, die dazu dient, sicherzustellen, dass das Nahtmaterial (40) nicht in die entgegengesetzte Richtung bewegt werden kann, insbesondere als "NO-GO"-Richtung bezeichnet, ohne eine Zugkraft aufzubringen, die typischerweise notwendig ist, um einen Nahtmaterialstrang (42) zu brechen, wobei die Flexarme (22) elastisch nach innen zusammenfallen, um die Verriegelungsverengungsöffnung (26) zu verengen.

14. Chirurgisches Nahtmaterialsystem nach einem der voranstehenden Ansprüche, wobei die Flexarmschlitze langgestreckt oval oder rennbahnförmig langgestreckt sind.

15. Chirurgisches Nahtmaterialsystem nach einem der voranstehenden Ansprüche, bei dem die Verriegelungsöffnung (14) der Geweberückhaltung / des Nahtmaterialfangs (10) auf der Austrittsseite der Verriegelungsöffnung (14) einen höheren Widerstand und auf der Eintrittsseite der Verriegelungsöffnung (14) einen geringeren Widerstand aufweist, vorzugsweise:
derart, dass das Nahtmaterial (40) leicht durch den Eintritt zu der Verriegelungsöffnung (14) der Geweberückhaltung / des Nahtmaterialfangs (10) hindurchtritt und durch die Austrittsseite der Verriegelungsöffnung (14) austritt, wobei beides die Vorwärtsbewegung des Nahtmaterials (40) ermöglicht, als "Go"-Richtung bezeichnet; und/oder
derart, dass verhindert wird, dass sich das Nahtmaterial (40) rückwärts bewegt oder von dem Eingang zu der Verriegelungsöffnung (14) zurückgezogen wird, was als "No-Go"-Richtung bezeichnet wird,
insbesondere:
derart, dass sich das Nahtmaterial (40) nicht durch die Verriegelungsöffnung (14) von der Austrittsseite zur Eintrittsseite ohne vollständiges Nahtmaterialversagen bewegen kann; und/oder
derart, dass die Kräfte in der "No-Go"-Richtung maximiert werden, während die Kräfte in der Vorwärtsrichtung minimiert werden, um einen Unterschied zu erzeugen.

## Revendications

1. Système de suture chirurgicale pour la réparation tissulaire et le rattachement de tissu déchiré à un substrat tissulaire ou à un implant médical, vétérinaire ou dentaire, le système comprenant une suture (40) souple allongée ayant une pluralité de protubérances (44) espacées longitudinalement sur une longueur de celle-ci,
une retenue de tissu/capture de suture (10) ayant une ouverture de verrouillage (14) centrale pour recevoir un élément de suture de la suture (40) la traversant pour un mouvement longitudinal de la suture (40) uniquement dans un sens vers l'avant à travers l'ouverture de verrouillage (14), et
une pluralité de bras flexibles (22) définie par des fentes radiales (16) adjacentes se prolongeant de manière radiale,
**caractérisé en ce**
**que** chacune des fentes radiales (16) se termine à son extrémité extérieure dans un trou de dégagement (18) de fente et
**que** chacun des bras flexibles (22) est en outre défini par une fente de bras flexible allongée (20) et
**que** chaque coin interne des fentes de bras flexibles (20) est formé à proximité de l'un des trous de dégagement (18) de fente, de sorte que l'espacement entre les trous de dégagement (18) et les coins internes influence sensiblement la flexibilité globale du bras flexible (20).

2. Système de suture chirurgicale selon la revendication 1, dans lequel le système comprend un implant, l'implant comprenant un corps implantable qui est configuré pour recevoir de manière sûre la retenue de tissu/capture de suture (10).

3. Système de suture chirurgicale selon la revendication 1 ou 2, dans lequel une entrée d'ouverture de verrouillage (24) de l'ouverture de verrouillage (14) centrale est sensiblement plus grande que chacune des protubérances (44) le long de la suture souple (40) afin de permettre l'insertion à partir divers angles de la suture souple (40) dans l'ouverture de verrouillage (14) centrale.

4. Système de suture chirurgicale selon l'une quelconque des revendications précédentes, comprenant en outre un ancrage de tissu ou un implant de prothèse (190) ayant une cavité pour y recevoir et fixer ladite retenue de tissu/capture de suture (10), la suture (40) pouvant être fixée à l'ancrage de tissu ou à l'implant de prothèse (190).

5. Système de suture chirurgicale selon l'une quelconque des revendications précédentes, dans lequel ladite retenue de tissu/capture de suture (10) comprend en outre :
une pluralité de fentes radiales (16) espacées se prolongeant chacune radialement vers l'extérieur de ladite ouverture de verrouillage (14) vers, mais pas jusqu'à, une périphérie d'un corps (12) de section ovale aplati de la retenue de tissu/capture de suture (10) pour définir un bras flexible (22) entre chacune des fentes radiales (16) adjacentes ; et une extrémité radialement la plus à l'intérieur de chacun desdits bras flexibles (22) définissant une ouverture de restriction de verrouillage (26) de l'ouverture de verrouillage (14).

6. Système de suture chirurgicale selon la revindication 5, dans lequel ladite retenue de tissu/capture de suture (10) comprend en outre :
une fente de bras flexible (20) formée à travers une partie médiane de chacun des bras flexibles (22) et qui est positionnée dans une relation régulièrement espacée entre une extrémité fermée de chacune des fentes radiales (16) adjacentes ; et/ou
les fentes de bras flexibles (20) pour commander la force requise pour tirer la suture (40) à travers l'ouverture de verrouillage (14) ; et/ou
l'ouverture de restriction de verrouillage (26), lorsque les bras flexibles (22) sont relâchés, qui a un diamètre sensiblement plus petit qu'un diamètre des protubérances (44) ; et/ou
l'ouverture de verrouillage (14) qui a une entrée d'ouverture de verrouillage (24) agrandie adjacente à un côté d'entrée de suture du corps (12) pour recevoir la suture (40) et permettre à la suture (40) de passer à travers et d'être poussée à travers le verrouillage ouverture de restriction (26) ; et/ou
l'ouverture de verrouillage (14) qui a une restriction inverse adjacente à un côté de sortie de suture dudit corps (12) pour empêcher que la suture (40), une fois qu'elle a été tirée à travers l'ouverture de verrouillage (14), de bouger dans une directement de mouvement inverse.

7. Système de suture chirurgicale selon l'une quelconque des revendications précédentes, dans lequel la retenue de tissu/capture de suture (10) chirurgicale comprend un corps comprenant une ouverture de verrouillage (14) dimensionnée pour recevoir une suture souple (40) allongée mince comportant une pluralité de protubérances (44) espacées longitudinalement le long d'une longueur de celle-ci traversant l'ouverture de verrouillage (14) pour empêcher de manière coopérative le mouvement longitudinal de la suture (40) uniquement dans un sens de mouvement vers l'avant à travers l'ouverture de verrouillage (14).

8. Système de suture chirurgicale selon la revendication 7, comprenant en outre un ancrage de tissu, une prothèse ou un dispositif implantable servant à un autre but que celui d'ancrage ayant une cavité pour recevoir et fixer ladite retenue de tissu/capture de suture (10), la suture (40) pouvant être fixée à l'ancrage de tissu ou à l'implant de prothèse (190).

9. Système de suture chirurgicale selon la revendication 7 ou 8, la retenue de tissu/capture de suture (10) comprenant en outre une pluralité de fentes radiales (16) espacées se prolongeant chacune radialement vers l'extérieur de ladite ouverture de verrouillage (14) vers, mais pas jusqu'à, une périphérie d'un corps de section ovale aplati pour définir un bras flexible (22) entre chacune des fentes radiales (16) adjacentes ; et une extrémité radialement la plus à l'intérieur de chacun desdits bras flexibles (22) définissant une ouverture de restriction de verrouillage (26) de l'ouverture de verrouillage (14).

10. Système de suture chirurgicale selon la revendication 9, comprenant en outre une fente de bras flexible (20) formée à travers une partie médiane de chacun des bras flexibles (22) et qui est positionnée dans une relation régulièrement espacée entre une extrémité fermée de chacune des fentes radiales (16) adjacentes ; de preference
les fentes de bras flexibles (20) pour commander la force requise pour tirer la suture (40) à travers l'ouverture de verrouillage (14) ; de preference
l'ouverture de restriction de verrouillage (26), lorsque les bras flexibles (22) sont relâchés, qui a un diamètre sensiblement plus petit qu'un diamètre des protubérances (44) ; et/ou
l'ouverture de verrouillage (14) qui a une entrée d'ouverture de verrouillage agrandie adjacente à un côté d'entrée de suture du corps (12) pour recevoir la suture (40) et permettre à la suture (40) de passer à travers et d'être poussée à travers le verrouillage ouverture de restriction (26) ; et/ou
l'ouverture de verrouillage (14) qui a une restriction inverse adjacente à un côté de sortie de suture dudit corps (12) pour empêcher que la suture (40), une fois qu'elle a été tirée à travers l'ouverture de verrouillage (14), de bouger dans une directement de mouvement inverse en réduisant la taille de l'ouverture de restriction de verrouillage (26) lorsque les bras flexibles (22) sont fléchis dans la direction inverse de la force appliquée à la suture (40).

11. Système de suture chirurgicale selon l'une quelconque des revendications précédentes, dans lequel l'ouverture de verrouillage (14) permet l'entrée de la suture (40) dans celle-ci avec une force minimale et une force maximisante empêchant le mouvement de la suture (40) dans la direction inverse, créant un rapport de force différentielle entre la force nécessaire pour déplacer la suture dans la direction inverse, appelée force "NO-GO", et la force nécessaire pour déplacer la suture dans la direction avant, appelée force "GO", dépassant un rapport de 1,5:1.

12. Système de suture chirurgicale selon l'une quelconque des revendications précédentes, dans lequel chacune des protubérances (44) est guidée par une approche de fente (32) et s'appuie contre le siège de tête de la suture, de sorte que l'application d'une pression supplémentaire de manière axiale à la suture (40) provoque le fléchissement vers l'extérieur du contour interne correspondant de chacun des bras flexibles (22) pour élargir la restriction d'ouverture de verrouillage jusqu'à ce que le nombre souhaité de protubérances (44) soit tiré à travers celle-ci, de préférence après quoi les bras flexibles (22) reviennent manière automatique et élastique à la position de repos, entraînant l'effondrement de la restriction d'ouverture de verrouillage.

13. Système de suture chirurgicale selon l'une quelconque des revendications précédentes, dans lequel l'extrémité de sortie de l'ouverture de verrouillage (14) de suture comprend une restriction inverse qui sert à assurer que la suture (40) ne peut pas être déplacée dans la direction opposée, en particulier dans la direction nommée "NO-GO", sans appliquer une force de traction typiquement nécessaire pour fracturer un fil de suture (42), les bras flexibles (22) s'effondrant élastiquement vers l'intérieur pour rétrécir l'ouverture de restriction de verrouillage (26).

14. Système de suture chirurgicale selon l'une quelconque des revendications précédentes, dans lequel les fentes de bras flexibles sont ovales allongées ou allongées en forme de circuit.

15. Système de suture chirurgicale selon l'une quelconque des revendications précédentes, dans lequel l'ouverture de verrouillage (14) de la retenue de tissu/capture de suture (10) fournit une résistance supérieure du côté de sortie de l'ouverture de verrouillage (14) et une résistance inférieure du côté d'entrée de l'ouverture de verrouillage (14), de préférence :
de sorte que la suture (40) passe facilement à travers l'entrée de l'ouverture de verrouillage (14) de la retenue de tissu/capture de suture (10) et quitte par le côté de sortie de l'ouverture de verrouillage (14), ce qui permet à la suture (40) de se déplacer vers la direction avant, nommée direction "Go"; et/ou
de sorte que la suture (40) ne puisse pas se déplacer en direction inverse ou être tirée vers l'arrière à partir de l'entrée de l'ouverture de verrouillage (14), nommée direction "No-Go",
en particulier :
de sorte que la suture (40) ne puisse pas se déplacer à travers l'ouverture de verrouillage (14) depuis le côté de sortie vers le côté d'entrée sans une rupture de suture complète ; et/ou
de sorte que les forces dans la direction "No-Go" soient maximisées, tout en minimisant les forces dans la direction avant pour créer un différentiel.
